# EUROPÄISCHE PATENTANMELDUNG

(11) **EP 4 838 246 A1**
(43) Veröffentlichungstag der Anmeldung: **11.11.2026**
(21) Anmeldenummer: 26176840.2
(22) Anmeldetag: 06.05.2026
(51) Int. Cl.: G01R 33/3875, A61B 5/055, G01N 24/08, G01R 33/46

(54) **VERFAHREN ZUR KORREKTUR EINES MAGNETISCHEN MESSSIGNALS**

(30) Priorität: 07.05.2025 DE 102025117403
(71) Anmelder: Bruker Biospin GmbH & Co. KG, 76275 Ettlingen (DE)
(72) Erfinder: Herbig, Alexander, 76135 Karlsruhe (DE); Sattler, Rainer, 8603 Schwerzenbach (CH)
(74) Vertreter: Altmann Stößel Dick Patentanwälte PartG mbB

(57) **Zusammenfassung**

Verfahren zur Ermittlung und Anwendung eines Korrektursignals auf eine Korrekturspule einer NMR-Apparatur zur Kompensation von zumindest einer Störung im Magnetfeld der NMR-Apparatur, insbesondere umfassend die Schritte:
h) Klassifizierung kritischer Punkte in Polstellen oder Sprungstellen,
I. wobei ein kritischer Punkt eines Teilsignals einer Polstelle entspricht, wenn die Absolutdifferenz des kritischen Punktes zu einem weiteren Punkt des entsprechenden Teilsignals in einem Suchintervall einer Breite w nach dem kritischen Punkt kleiner als das k-fache der korrigierten Standardabweichung ist,
II. wobei ein kritischer Punkt einer Sprungstelle des Teilsignals zuentspricht, wenn die Absolutdifferenz des kritischen Punktes zu allen Punkten des entsprechenden Teilsignals in dem Suchintervall der Breite w nach dem kritischen Punkt größer als das k-fache der korrigierten Standardabweichung ist,

i) Korrektur aller Punkte in einem Polstellenbereich, die auf eine Polstelle folgen und im Suchintervall der Breite w eine Absolutdifferenz zum kritischen Punkt haben, die größer als das k-fache der korrigierten Standardabweichung ist, durch Interpolation mit zu dem Polstellenbereich benachbarten Werten des Teilsignals,
j) Korrektur einer Sprungstelle durch Subtraktion einer Sprunghöhe von allen auf die Sprungstelle folgenden Werten des Teilsignals, wobei die Sprunghöhe aus den Werten des Teilsignals vor und nach der Sprungstelle ermittelt wird,
k) Mittelung aller durch die vorhergehenden Schritte korrigierten Teilsignale, wobei ein Korrektursignal erzeugt wird,
l) Anwendung des Korrektursignals aus Schritt k) auf eine Korrekturspule der NMR-Apparatur zur Kompensation der zumindest einen Störung im Magnetfeld der NMR-Apparatur.

## Beschreibung

### Technisches Gebiet

Die Erfindung betrifft ein Verfahren zur Ermittlung und Anwendung eines Korrektursignals auf eine Korrekturspule einer NMR-Apparatur zur Kompensation von zumindest einer Störung im Magnetfeld der NMR-Apparatur. Das technische Gebiet der Erfindung liegt im Bereich der Kernspinresonanz (NMR), einer Technik, die zur Analyse der molekularen Struktur von Materialien und biologischen Körpern eingesetzt werden kann.

### Technischer Hintergrund

NMR-Magnete können eine geringfügige Drift des Hauptmagnetfeldes aufweisen, die auf verschiedene Ursachen zurückzuführen ist. Diese Drift kann durch Veränderungen der Umgebungsbedingungen, wie Luftdruck und Temperatur, oder durch interne magnetische Effekte verursacht werden. Zur Stabilisierung des Hauptmagnetfeldes wird der Fluss durch die Magnetspule mittels einer Feld-Kompensationsspule angepasst. Gleichzeitig erfolgt in der Regel eine Homogenisierung des Hauptmagnetfeldes im Messvolumen durch eine Autoshim-Vorrichtung. Die aktive Feldstabilisierung und -Homogenisierung kann aufgrund von Schaltvorgängen in den eingesetzten Spulen und der damit verbundenen Feldstabilisierungselektronik zu Störsignalen im Hauptmagnetfeld führen.

Störungen im Magnetfeld, die durch die automatische Feldstabilisierung hervorgerufen werden, können nur unter bestimmten Bedingungen kompensiert werden. Diese Bedingungen können erfordern, dass der Probenkopf, das Sample und die NMR-Apparatur die Anwendung eines Feld-Locks zulassen. Diese Einschränkung kann eine erhebliche Begrenzung für die Gesamtheit der experimentellen NMR-Methoden und Anwendungen darstellen. Beispielsweise gibt es bei Festkörper-NMR kein Feld-Lock, wodurch die Kompensation der beschriebenen Störungen bei Festkörperexperimenten bisher nicht möglich ist.

EP2246709A1 beschreibt eine Vorrichtung, die einen HF-Generator, ein NMR-Sende- und Empfangssystem sowie einen ersten Regelkreis umfasst, mit dem die Frequenz fRF des HF-Generators mit der Resonanzfrequenz f0 einer NMR-Linie synchronisiert wird. Es wird hierbei aus dem Signal des HF-Generators eine Folge von Anregungsimpulsen der Repetitionsfrequenz fm erzeugt, mit der Kernspins einer bestimmten Resonanzfrequenz einer zugehörigen NMR-Linie quasi-kontinuierlich (CW) angeregt werden. In den Zeiten zwischen den Anregungsimpulsen wird das NMR-Signal empfangen, wobei die Periodenzeit 1/f m viel kleiner als die Relaxationszeit der NMR-Linie gewählt wird (vorzugsweise kürzer als 1/10 der Relaxationszeit). Das in den Niederfrequenz-Bereich hinunter gemischte NMR-Signal UD wird dazu benutzt, mit Hilfe des ersten Regelkreises den Wert der Sendefrequenz (=Frequenzlock) oder den Wert des B0 -Feldes (=Feldlock) so zu regeln, dass Frequenz und Phase des HF-Generators und der NMR-Linie möglichst genau übereinstimmen. Dies geschieht, indem ein Phasenschieber, der die Hochfrequenzphase des NMR-Sende- und Empfangssystems im ersten Regelkreis variiert, von einem zweiten Regelkreis gesteuert wird, dessen Eingangssignal aus einer Signalextraktionsstufe kommt, die den sägezahnförmigen AC-Anteil des Signals UD extrahiert und der dieses Eingangssignal auf Null regelt. Damit lässt sich der Einfluss der HF-Phase auf den Synchronisationsvorgang berücksichtigen, um dadurch die Genauigkeit, mit der auf die Resonanzfrequenz einer NMR-Linie synchronisiert wird, zu verbessern.

EP0522191B1 beschreibt ein Verfahren zum Kompensieren von zeitvarianten Feldstörungen in Magnetfeldern von Elektromagneten mit hoher Feldhomogenität, insbesondere in Probenräumen von supraleitenden Elektromagneten für Messungen magnetischer Resonanz. Dabei wird das Dispersionssignal ux des Kernsignals einer Vergleichssubstanz aufgenommen und zur Kompensation durch Erzeugen eines von dem Dispersionssignals abhängigen Stroms in einer Feldkorrekturspule des Elektromagneten herangezogen. Das Verfahren ist dadurch gekennzeichnet, dass zusätzlich das Absorptionssignal uy der Vergleichssubstanz aufgenommen wird, und dass die Kompensation in Abhängigkeit von der Größe ux/uy und/oder (1/uy)(dux/dt) vorgenommen wird. Diese sind weitgehend unabhängig von zahlreichen Einflussgrößen.

EP1202055A2 beschreibt eine Magnetresonanztomographie-Vorrichtung, die eine Schaltung zur Kompensation von magnetischen Störungen darin aufweist. Die Kompensationsschaltung umfasst einen Sensor zum Erfassen von Magnetfeldänderungen und zum Erzeugen eines Magnetfeldänderungssignals. Der Sensor ist mit einer Steuerschaltung gekoppelt, für die eine Übertragungsfunktion vorhanden ist. Die Steuerschaltung wird verwendet, um ein Kompensationssignal als Reaktion auf das Magnetfeldänderungssignal zu erzeugen. Das Kompensationssignal kann mit den Kompensationsspulen gekoppelt werden, die sich am ersten Magnetteil und am zweiten Magnetteil der Vorrichtung befinden. Außerdem kann das Kompensationssignal eine zeitabhängige Vorspannung erzeugen oder die Mittenfrequenz ändern, um magnetische Störungen zu beseitigen.

DE 100 32 345 A1 beschreibt ein Verfahren zum Korrigieren von Störflüssen auf die durch einen oder mehrere Hochfrequenz-Anregungsimpulse angeregten Magnetresonanz-Signale einer im Messvolumen einer MR-Apparatur angeordneten Substanz.

### Aufgabe der Erfindung

Es ist eine Aufgabe der Erfindung ein Verfahren zur Ermittlung und Anwendung eines Korrektursignals auf eine Korrekturspule einer NMR-Apparatur zur Kompensation von zumindest einer Störung im Magnetfeld der NMR-Apparatur bereitzustellen, welches die Nachteile des Stands der Technik zumindest weitgehend vermeidet.

Insbesondere soll die Erfindung die Variation der Drift des Hauptmagnetfeldes, die durch externe und interne Faktoren verursacht wird, identifizieren und korrigieren, um die Qualität der NMR-Daten zu verbessern.

Weiterhin soll insbesondere die Erfindung die Kontinuität und Präzision der Messungen gewährleisten, indem spezifische Korrekturmethoden angewendet werden.

Weiterhin soll insbesondere die Erfindung die Analyse von Materialien und biologischen Körpern optimieren, indem sie die Stabilität des Messsignals sicherstellt.

### Allgemeine Beschreibung der Erfindung

Diese oben beschriebene Aufgabe wird gelöst durch ein Verfahren zur Ermittlung und Anwendung eines Korrektursignals auf eine Korrekturspule einer NMR-Apparatur zur Kompensation von zumindest einer Störung im Magnetfeld der NMR-Apparatur mit den Merkmalen der unabhängigen Patentansprüche. Vorteilhafte Weiterbildungen, welche einzeln oder in beliebiger Kombination realisierbar sind, sind in den abhängigen Ansprüchen dargestellt.

In einem ersten Aspekt wird ein Verfahren zur Ermittlung und Anwendung eines Korrektursignals auf eine Korrekturspule einer NMR-Apparatur zur Kompensation von zumindest einer Störung im Magnetfeld der NMR-Apparatur offenbart. Für diesen Aspekt kann auf jeden weiteren Aspekt, jede Definition und/oder jede Ausführungsform wie hierin beschrieben verwiesen werden.

Der Begriff "Korrektur", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf die Anpassung oder Verbesserung eines oder mehrere Signale oder Messungen beziehen, um zumindest einen Fehler oder zumindest eine Unregelmäßigkeit zu beseitigen. Im Kontext der vorliegenden Erfindung kann sich "Korrektur" auf einen Prozess beziehen, der angewendet wird, um zumindest eine Störung in zumindest einem magnetischen Messsignal einer NMR-Apparatur zu identifizieren und zu beheben. Die Korrektur kann die Ermittlung eines oder mehrerer kritischer Punkte umfassen, die als Polstellen oder Sprungstellen klassifiziert werden, und die Anwendung spezifischer Methoden zur Beseitigung oder Kompensation des Einflusses derselben auf das zumindest eine magnetische Messsignal. Die Korrektur kann die Kontinuität und die Genauigkeit des einen magnetischen Messsignals gewährleisten.

Der Begriff "NMR-Apparatur", mithin "Nuclear Magnetic Resonance - Apparatur", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung zur Durchführung von Kernspinresonanz beziehen. Die NMR-Apparatur kann dazu ausgelegt sein, magnetische Messsignale zu erfassen oder zu generieren, die für die Analyse von Materialien oder biologischen Körpern und deren molekularen Strukturen verwendet werden. Die Abkürzung NMR kann dabei für ,Nuclear Magnetic Resonance' oder Kernspinresonanz stehen.

Der Begriff "Störung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine unerwünschte Abweichung und/oder Unterbrechung im normalen Verlauf eines Signals oder Prozesses beziehen. Die zumindest eine Störung kann durch einen oder mehrere externe Faktoren wie eine Temperaturänderung, eine mechanische Vibration oder eine elektromagnetische Interferenz verursacht werden. Insbesondere, wenn das Magnetfeld und damit auch das magnetische Messignal mittels zumindest eines supraleitenden Magnetes erzeugt wird, können Störungen zusätzlich durch einen oder mehrere interne Faktoren wie eine Veränderung einer Position oder einer Struktur zumindest eines Flussschlauchs im supraleitenden Magneten oder zumindest einer Veränderung im Magnetfeld aufgrund von Materialeigenschaften des supraleitenden Magneten verursacht werden. Die zumindest eine Störung kann die Genauigkeit und Zuverlässigkeit der Messungen der NMR-Apparatur beeinträchtigen.

Das Verfahren umfasst die nachfolgend beschriebenen Schritte. Die beschriebenen Schritte können bevorzugt in der benannten Reihenfolge durchgeführt werden. Auch eine andere Reihenfolge ist jedoch grundsätzlich möglich. Weiterhin ist es möglich, dass zwei oder mehr der Verfahrensschritte zeitlich überlappend oder gleichzeitig durchgeführt werden. Weiterhin ist es möglich, dass einer oder mehrere der Verfahrensschritte einfach oder auch mehrfach wiederholt durchgeführt werden. Das Verfahren kann weitere Verfahrensschritte umfassen, welche nicht dargestellt sind. Insbesondere kann bei einem supraleitenden Magneten zumindest ein externer Faktor, wie die periodische Aktivierung von Feldstabilisierungselektronik zu zumindest einer wiederkehrenden Störung führen.

Das Verfahren umfasst den Schritt:
a) Unterteilung eines magnetischen Messsignals in eine Vielzahl von Teilsignalen, wobei das eine magnetische Messsignal in sich wiederholenden Störperioden gleicher Länge *T* zumindest eine Störung aufgrund der zumindest einen Störung im Magnetfeld der NMR-Apparatur erfährt, wobei jedes Teilsignal dem einen magnetischen Messsignal in einer Störperiode entspricht.

Der Begriff "magnetisches Messsignal", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf ein Signal beziehen, das durch die Wechselwirkung von zumindest einem Magnetfeld mit einer Probe, mithin einem Material oder einem biologischen Körper, mittels einer NMR-Apparatur erzeugt wird. Das magnetische Messsignal kann Informationen oder Daten über die Struktur und/oder zumindest eine Eigenschaft der untersuchten Probe umfassen. Jede Messung des magnetischen Messsignals kann in äquidistanten Zeitschritten zwischen 150 Millisekunden (ms) und 600 ms erfolgen.

Der Begriff "Teilsignal", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen Abschnitt oder ein Segment eines größeren Signals beziehen. Ein Teilsignal kann beispielsweise derart ausgelegt sein, dass es spezifische Informationen oder Merkmale eines magnetischen Messsignals repräsentiert. Die Unterteilung eines magnetischen Messsignals in Teilsignale kann durch zumindest eine Maßnahme erfolgen, insbesondere wobei die Maßnahme zumindest einen der folgenden Schritte umfasst: Segmentierung des Signals basierend auf Zeitintervallen, Frequenzbereichen oder Amplitudenwerten. Ein oder mehrere Teilsignale können genutzt werden, um die Analyse und Verarbeitung von Daten zu erleichtern, indem sie die Identifikation von Störungen oder Unregelmäßigkeiten innerhalb des größeren Signals ermöglichen. Jedes Teilsignal entspricht dabei einem Teil des einen magnetischen Messsignals in einer Störperiode. Das Teilsignal kann Punkte, mithin Messpunkte, aufweisen.

Der Begriff "Störperiode", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf ein wiederkehrendes Zeitintervall beziehen, in dem ein Signal durch zumindest eine Störung beeinflusst wird. Die Störperiode kann in regelmäßigen Abständen auftreten und die Messungen der NMR-Apparatur stören. Im Kontext der Erfindung kann eine Störperiode als kritischer Zeitraum betrachtet werden, in denen Korrekturmaßnahmen erforderlich sind, um die Stabilität des Messsignals zu gewährleisten. Typischerweise ist die Länge *T* der Störperioden zwischen 250 Sekunden (s) und 5000 s.

Nach Schritt a), und bevorzugt vor Schritt b), kann eine Offsetkorrektur für jedes Teilsignal erfolgen, so dass die Werte der letzten Punkte der Teilsignale übereinstimmen, insbesondere wobei die Offsetkorrektur durch Subtraktion des Wertes des letzten Punktes vom entsprechenden gesamten Teilsignal erfolgt.

Der Begriff "Offsetkorrektur", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf die Anpassung zumindest eines Signals beziehen, um eine oder mehrere systematische Abweichungen oder Verschiebungen zu beseitigen. Eine Offsetkorrektur kann durch zumindest eine mathematische Anpassung erfolgen, insbesondere wobei die Anpassung zumindest eine der folgenden Maßnahmen umfasst: Subtraktion eines konstanten Wertes vom gesamten Signal, Anpassung der Signalbasislinie oder Kalibrierung des Signals auf einen Referenzwert. Eine oder mehrere Offsetkorrekturen können beispielsweise derart ausgelegt sein, dass sie die Genauigkeit und Zuverlässigkeit eines magnetischen Messsignals verbessern.

Das Verfahren umfasst weiterhin den Schritt:
b) Ermittlung eines Glättungssignals für jedes Teilsignal aus dem Teilsignal.

Der Begriff "Glättungssignal", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf ein Signal beziehen, das durch die Glättung oder Filterung eines ursprünglichen Signals erzeugt wird, um Rauschen oder Unregelmäßigkeiten zu reduzieren. Ein Glättungssignal kann beispielsweise derart ausgelegt sein, dass es die wesentlichen Merkmale eines magnetischen Messsignals beibehält, während es Störungen minimiert. Die Erzeugung eines Glättungssignals kann durch zumindest eine Maßnahme erfolgen, insbesondere wobei die Maßnahme zumindest eine der folgenden Techniken umfasst: Anwendung eines gleitenden Mittelwerts, Einsatz eines Tiefpassfilters zum Beispiel mittels Durchführung einer Fourier-Transformation. Bevorzugt kann jedes Glättungssignal durch einen Tiefpass-Filter nach einer Fouriertransformation ermittelt werden. Ein oder mehrere Glättungssignale können genutzt werden, um die Analyse und Verarbeitung von Daten zu erleichtern, indem sie eine klarere Darstellung der zugrunde liegenden Signalstruktur bieten.

Jedes Glättungssignal kann durch einen gleitenden Mittelwert mit einer Fensterbreite *F* über jedes Teilsignal ermittelt werden, wobei die Fensterbreite *F* kleiner sein kann als die Länge *T* der Störperiode, insbesondere wobei die Fensterbreite *F* zwischen 10 s und 100 s groß ist.

Der Begriff "Fensterbreite" bezieht sich auf die Breite eines Zeitintervalls, das bei der Ermittlung eines Glättungssignals durch einen gleitenden Mittelwert verwendet wird. Die Fensterbreite ist derart ausgelegt, dass sie die Glättung des magnetischen Messsignals optimiert, indem sie die Auswirkungen von Störungen minimiert und die Genauigkeit der Messungen verbessert.

Das Verfahren umfasst weiterhin den Schritt:
c) Berechnung einer Standardabweichung für jedes Teilsignal von dessen Glättungssignal.

Der Begriff "Standardabweichung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf ein statistisches Maß beziehen, das die Streuung oder Variation einer Menge von Werten um ihren Mittelwert beschreibt. Die Standardabweichung kann beispielsweise derart ausgelegt sein, dass sie die Abweichungen der Messwerte eines Teilsignals von einem Glättungssignal quantifiziert. Die Berechnung der Standardabweichung kann durch zumindest eine mathematische Operation erfolgen. Im Rahmen dieser Offenbarung ist die Standardabweichung σ*_{G}* typischerweise gegeben durch die Gleichung ${σ}_{G} = \sqrt{\frac{1}{{N}_{t}} {\sum }_{t} {\left({H}_{0}\left(t\right)-{\left〈{H}_{0}\right〉}_{G}\left(t\right)\right)}^{2}}$ (1), wobei *H*₀(*t*) den Messwerten eines Teilsignals, 〈*H*₀〉*_{G}*(*t*) dem zugehörigen Glättungssignal, das in Schritt b) für das Teilsignal erzeugt wurde, und *Nₜ* der Anzahl der berücksichtigen Punkte des Teilsignal entspricht.

Die Standardabweichung kann, bevorzugt, unter Berücksichtigung aller Messwerte eines Teilsignals ermittelt werden. Dabei kann die Standardabweichung über eine Fensterbreite *F* ermittelt werden, wobei die Fensterbreite *F* kleiner ist als die Länge *T* der Störperiode, insbesondere wobei die Fensterbreite zwischen 10 s und 100 s ist.

Das Verfahren umfasst weiterhin den Schritt:
d) Bestimmung eines Konfidenzintervalls für alle Punkte jedes Teilsignals unter Verwendung des Glättungssignals und einem k-fachen der ermittelten Standardabweichung des entsprechenden Teilsignals, wobei k eine positive rationale Zahl größer oder gleich 1 ist.

Der Begriff "Konfidenzintervall", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen Bereich beziehen, der die Unsicherheit oder Variabilität einer Schätzung oder Messung quantifiziert. Ein Konfidenzintervall kann beispielsweise derart ausgelegt sein, dass es die Grenzen angibt, innerhalb derer ein bestimmter Wert mit einer bestimmten Wahrscheinlichkeit liegt. Die Bestimmung des Konfidenzintervalls *H*_{0*,*±}(*t*) kann mittels der Gleichung (2) ${H}_{0 , \pm } \left(t\right) = {\left〈{H}_{0}\right〉}_{G} \left(t\right) \pm k ⋅ {σ}_{G}$ (2) erfolgen, wobei σ*_{G}* der Standardabweichung entspricht, die in Schritt c) ermittelt wurde, wobei k ein Faktor ist, der die Größe des Konfidenzintervalls über die Standardabweichung hinaus angibt, und 〈*H*₀〉*_{G}*(*t*) dem Glättungssignal, das in Schritt b) für das Teilsignal erzeugt wurde, entspricht. Eine Größe des Konfidenzintervalls kann sich aus dem k-fachen der Standardabweichung bestimmen oder ihm entsprechen, wobei k, typischerweise, zwischen 2 und 20 liegt, insbesondere wobei das Konfidenzintervall das Glättungssignal 〈*H*₀〉*_{G}*(*t*) umgibt.

Das Verfahren umfasst weiterhin den Schritt:
e) Ermittlung kritischer Punkte jedes Teilsignals, wobei die kritischen Punkte die Punkte sind, die außerhalb des Konfidenzintervalls liegen.

Zur Ermittlung kritischer Punkte jedes Teilsignals kann ein Punkt des Teilsignals einem kritischen Punkt zugeordnet werden, wenn der Punkt außerhalb des Konfidenzintervalls liegt.

Somit kann ein Punkt *H*₀(*tᵢ*) als kritischer Punkt klassifiziert werden, wenn gilt $\left|{H}_{0}\left({t}_{i}\right)-{\left〈{H}_{0}\right〉}_{G}\left({t}_{i}\right)\right| > k ⋅ {σ}_{G}$ (3).

Andernfalls kann ein Punkt als nicht kritischer Punkt, mithin unkritischer Punkt, klassifiziert werden.

Das Verfahren weiterhin den Schritt:
f) Berechnung einer korrigierten Standardabweichung wie unter Schritt c) aber mit Auslassung aller kritischen Punkte aus Schritt e).

Typischerweise ist die korrigierte Standardabweichung σ*_{G,A}* gegeben durch die Gleichung ${σ}_{G , A} = \sqrt{\frac{1}{{N}_{t}} {\sum }_{t ∉ K , P} {\left({H}_{0}\left(t\right)-{\left〈{H}_{0}\right〉}_{G}\left(t\right)\right)}^{2}}$ (4), wobei *H*₀(*t*) der Messkurve eines Teilsignals, 〈*H*₀〉*_{G}*(*t*) dem Glättungssignal, das in Schritt b) für das Teilsignal erzeugt wurde, und *Nₜ* der Anzahl der berücksichtigen Punkte des Teilsignals entspricht. Der Index *t* ∉ *K, P* deutet an, dass alle kritischen Punkte aus Schritt e) nicht bei der Ermittlung der korrigierten Standardabweichung σ*_{G,A}* berücksichtigt werden.

Das Verfahren umfasst weiterhin den Schritt:
g) Erneute Ermittlung der kritischen Punkte jedes Teilsignals unter Berücksichtigung aller Punkte jedes Teilsignals, wobei die kritischen Punkte die Punkte sind, bei denen die Absolutdifferenz zwischen einem Punkt und seinem folgenden Punkt des entsprechenden Teilsignals größer ist als ein k-faches der korrigierten Standardabweichung.

Für die erneute Ermittlung der kritischen Punkte kann zunächst für jeden Punkt *H*₀(*tᵢ*) des Teilsignals eine, bevorzugt absolute, Differenz D wie folgt berechnet werden $D = \left|{H}_{0}\left({t}_{i + 1}\right)-{H}_{0}\left({t}_{i}\right)\right|$ (5).

Dabei ist *H*₀(*t*_{*i+*1}) der folgende Punkt. Ein Punkt *H*₀(*t*_{*i+*1}) kann als kritischer Punkt klassifiziert werden, wenn gilt *D > k ·* σ*_{G,A}.* Andernfalls kann ein Punkt *H*₀(*t*_{*i*+1}) als nicht kritischer Punkt, mithin unkritischer Punkt, klassifiziert werden.

Der Begriff "Absolutdifferenz" bezieht sich auf den Betrag der Differenz der angegebenen Größen. *k* kann eine positive rationale Zahl größer oder gleich 1 sein, insbesondere kann k gleich 1 sein. k liegt vorzugsweise zwischen 2 und 20.

Das Verfahren umfasst weiterhin den Schritt:
h) Klassifizierung der kritischen Punkte in Polstellen oder Sprungstellen,
I. wobei ein kritischer Punkt einer Polstelle entspricht, wenn die Absolutdifferenz des kritischen Punktes zu einem weiteren Punkt des entsprechenden Teilsignals in einem Suchintervall einer Breite *w* nach dem kritischen Punkt kleiner als das k-fache der korrigierten Standardabweichung ist,
II. wobei ein kritischer Punkt einer Sprungstelle entspricht, wenn die Absolutdifferenz des kritischen Punktes zu allen Punkten des entsprechenden Teilsignals in dem Suchintervall der Breite w nach dem kritischen Punkt größer als das k-fache der korrigierten Standardabweichung ist.

Der Begriff "Polstelle", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen Punkt in einem Signal beziehen, an dem eine abrupte Änderung oder Unregelmäßigkeit auftritt. Eine Polstelle kann beispielsweise derart ausgelegt sein, dass sie eine Feldänderung im magnetischen Messsignal darstellt, die innerhalb eines vergleichsweise kleinen Zeitintervalls auftritt. Der Begriff "Sprungstelle", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen Punkt in einem Signal beziehen, an dem eine plötzliche und dauerhafte Änderung des Signalwerts auftritt. Eine Sprungstelle kann beispielsweise derart ausgelegt sein, dass sie eine Feldänderung im magnetischen Messsignal darstellt, die nicht innerhalb eines kurzen Zeitintervalls zurückkehrt.

Wenn ein Punkt *H*₀(*tᵢ*) als kritischer Punkt in Schritt g) ermittelt wird, mithin wenn $\left|{H}_{0}\left({t}_{i + 1}\right)-{H}_{0}\left({t}_{i}\right)\right| > k ⋅ {σ}_{G , A}$ gilt, dann kann für diesen Punkt *H*₀(*tᵢ*) ermittelt werden, ob es innerhalb eines Suchintervalls der Breite w einen weiteren Punkt *H*₀(*tⱼ*) gibt, der innerhalb des Konfidenzintervalls liegt, mithin ob es einen weiteren Punkt *H*₀(*tⱼ*) gibt, für den gilt $\left|{H}_{0}\left({t}_{j}\right)-{H}_{0}\left({t}_{i}\right)\right| \leq k ⋅ {σ}_{G , A}$ wobei ${t}_{i} < {t}_{j} < {t}_{i} + w .$

Gibt es einen weiteren Punkt *H*₀(*tⱼ*) der die Gleichung erfüllt, dann wird der kritische Punkt als Polstelle klassifiziert. Gibt es keinen weiteren Punkt *H*₀(*tⱼ*) der die Gleichung erfüllt, dann wird der kritische Punkt als Sprungstelle klassifiziert. Alle Punkte, die innerhalb des Sucherintervalls der Breite w auf eine Polstelle folgen und eine Absolutdifferenz größer als die k-fache korrigierte Standardabweichung zum kritischen Punkt der Polstelle haben, werden dem zugehörigen Polstellenbereich zugeordnet.

Die Breite w des Suchintervalls kann kleiner sein als die Länge *T* der Störperiode, insbesondere wobei die Breite w des Suchintervalls zwischen 1 s und 30 s liegt.

In Schritt h)II. kann beidseitig der Sprungstelle die Krümmung des Teilsignals ermittelt werden und das Teilsignal kann von dem Korrekturverfahren ausgeschlossen werden, wenn die Krümmung des Teilsignals an der Sprungstelle ihr Vorzeichen wechselt.

Das Verfahren umfasst weiterhin den Schritt:
i) Korrektur aller Punkte in einem Polstellenbereich, die auf eine Polstelle folgen und im Suchintervall der Breite w eine Absolutdifferenz zum kritischen Punkt haben, die größer als das k-fache der korrigierten Standardabweichung ist durch Interpolation mit zu dem Polstellenbereich benachbarten Werten des Teilsignals.

Der Begriff "Korrektur aller Punkte in einem Polstellenbereich", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf die Anpassung, Hebung oder Beseitigung von Unregelmäßigkeiten in einem Signal beziehen, die durch einen Polstellenbereich verursacht werden. Korrektur kann durch zumindest eine mathematische Operation erfolgen, insbesondere wobei die mathematische Operation zumindest einen der folgenden Schritte umfasst: Interpolation der Signalwerte unter Berücksichtigung der benachbarten Werte des Teilsignals.

Der Begriff "Interpolieren", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf die Schätzung von Werten innerhalb des Bereichs der bekannten Datenpunkte beziehen. Interpolieren kann durch zumindest eine mathematische Methode erfolgen, insbesondere wobei die Methode zumindest eine der folgenden Maßnahmen umfasst: Anwendung von linearen Interpolationsverfahren, Nutzung von polynomialen Interpolationsfunktionen oder Einsatz von Spline-Methoden zur Interpolation der Daten. Interpolieren kann insbesondere dazu dienen, die Kontinuität eines Signals zu gewährleisten, indem es zumindest eine Lücke zwischen bekannten Datenpunkten füllt.

Typischerweise kann die Messkurve eines Teilsignals *H*₀(*t*) mittels Orthogonalpolynomen φ*ᵢ*(*t*) interpoliert werden. Dabei wird die Messkurve *H*₀(*t*) des Teilsignals folgendermaßen entwickelt: ${H}_{0} \left(t\right) = {\sum }_{k = 0}^{N} {a}_{k} {φ}_{k} \left(t\right) ,$ wobei ${a}_{k} = \int dt {H}_{0} \left(t\right) {φ}_{k}^{∗} \left(t\right)$ den Entwicklungskoeffizienten der Orthogonalpolynome *φᵢ*(*t*) entsprechen. Typischerweise wird die Entwicklung bei der Ordnung N = 80 abgebrochen. Zusätzlich können Chebyshev-Polynome verwendet werden.

Das Verfahren umfasst weiterhin den Schritt:
j) Korrektur einer Sprungstelle durch Subtraktion einer Sprunghöhe von allen auf die Sprungstelle folgenden Werten des Teilsignals, wobei die Sprunghöhe aus den Werten des Teilsignals vor und nach der Sprungstelle ermittelt wird.

Der Begriff "Korrektur einer Sprungstelle", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf die Anpassung, Hebung oder Beseitigung von Unregelmäßigkeiten in einem Signal beziehen, die durch eine Sprungstelle verursacht werden. Die Korrektur kann durch zumindest eine mathematische Operation erfolgen, insbesondere wobei die mathematische Operation zumindest einen der folgenden Schritte umfasst: Subtraktion einer Sprunghöhe von allen auf die Sprungstelle folgenden Werten des Teilsignals, Ermittlung der Sprunghöhe aus den Werten des Teilsignals vor und nach der Sprungstelle und Anpassung des Signalverlaufs, um die Kontinuität zu gewährleisten.

In Schritt j), können die Werte des Teilsignals innerhalb eines Sicherheitsintervalls mit einer Breite r um die Sprungstelle extrapoliert werden, und die Sprunghöhe aus der Differenz zwischen dem extrapolierten Teilsignal am Rand des Sicherheitsintervalls und dem Teilsignal an dieser Stelle ermittelt werden.

Der Begriff "Sicherheitsintervall", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen definierten Zeitbereich beziehen, der dazu dient, die Integrität und Zuverlässigkeit von Messdaten zu gewährleisten. Ein Sicherheitsintervall kann beispielsweise derart ausgelegt sein, dass es die Daten innerhalb eines bestimmten Zeitrahmens berücksichtigt und die Auswirkungen von Störungen minimiert.

Der Begriff "Extrapolieren", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf die Schätzung von Werten außerhalb des Bereichs der bekannten Datenpunkte beziehen. Extrapolieren kann durch zumindest eine mathematische Methode erfolgen, insbesondere wobei die Methode zumindest eine der folgenden Maßnahmen umfasst: Anwendung einer linearen Fortsetzung basierend auf vorhandenen Datenpunkten, Nutzung von polynomialen Funktionen zur Vorhersage von Trends oder Einsatz von statistischen Modellen zur Schätzung zukünftiger Werte. Ein oder mehrere extrapolierte Werte können genutzt werden, um die Analyse und Verarbeitung von Daten zu erleichtern, indem sie Vorhersagen über zukünftige Entwicklungen ermöglichen.

Entweder der Teil vor oder nach dem Sprung des Teilsignals kann extrapoliert werden, und die Sprunghöhe kann aus der Differenz zwischen dem extrapolierten Teil des Teilsignals am Rand des Sicherheitsintervalls *r* und dem anderen Teil des Teilsignals an dieser Stelle ermittelt werden. . Korrektursignals erfolgen, das derart ausgelegt ist, dass es den Einfluss zumindest einer Störung im magnetischen Messsignal kompensiert. Das Korrektursignal wird verwendet, um die Korrekturspule so zu steuern, dass sie den Einfluss der zumindest einen Störung im zumindest einen magnetischen Messsignal kompensiert.

In Schritt 1), kann das Korrektursignal für die Korrekturspule invertiert werden, um der zumindest einen Störung im magnetischen Messsignal entgegenzuwirken.

Der Begriff "invertieren", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf die Umkehrung oder Spiegelung eines Signals oder einer Funktion beziehen. Invertieren kann durch zumindest eine mathematische Operation erfolgen, insbesondere wobei die Operation zumindest einen der folgenden Schritte umfasst: Multiplikation des Signals mit -1, Spiegelung des Signals um seine Achse oder Phasenumkehr des Signals. Das invertierte Signal kann beispielsweise derart ausgelegt sein, dass es den Einfluss der zumindest einen Störung im magnetischen Messsignal kompensiert. Alternativ oder zusätzlich kann die Invertierung genutzt werden, um die Richtung eines Signals zu ändern, um eine gewünschte Korrektur zu erzielen. Die Invertierung kann darauf abzielen, die Genauigkeit und Zuverlässigkeit der Messungen zu verbessern.

Zumindest ein Teil der Verfahrensschritte kann mit zumindest einem der folgenden: einem verschiedenen *k,* einem verschiedenen Suchintervall einer Breite w, insbesondere mit verschiedenen Parametern *k* und *w*, wiederholt durchgeführt werden. Beispielsweise können in einem ersten Schritt alle Polstellen mit *k* = 10 und *w* = 30 s korrigiert werden. In einem zweiten Schritt können alle Sprungstellen mit *k* = 5 und w = 2 s korrigiert werden. In einem dritten Schritt können verbliebene Polstellen mit *k* = 5 und *w* = 5 s korrigiert werden. In einem vierten Schritt kann das korrigierte Teilsignal von der Berechnung des Korrektursignals in Schritt k) ausgeschlossen werden, wenn mit *k* = 15 und *w* = 1 s noch Polstellen oder Sprungstellen vorhanden sind.

Das Verfahren kann ein computer-implementiertes Verfahren sein. Insbesondere die Verfahrensschritte a) bis k) können von einem Computer durchgeführt werden, wobei der Verfahrensschritt 1) mittels der Korrekturspule der NMR-Apparatur durchgeführt werden kann. Weitere Verfahrensschritte, die hier nicht explizit ausgeführt sind, aber die mittels eines Computers durchgeführt werden können, können Teil des computer-implementierten Verfahrens sein.

Der Begriff "computer-implementiert", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf einen Prozess beziehen, welcher vollständig oder teilweise unter Verwendung eines Computers implementiert ist, insbesondere unter Verwendung mindestens eines Prozessors. Der Begriff "computer-implementiert" kann sich vorzugsweise also auf ein Verfahren beziehen, das mindestens einen Computer und/oder mindestens ein Computernetz einbindet. Der Computer und/oder das Computernetzwerk kann mindestens einen Prozessor umfassen, der zur Durchführung mindestens eines der Verfahrensschritte des Verfahrens ausgebildet ist. Vorzugsweise wird jeder der Verfahrensschritte durch den Computer und/oder das Computernetzwerk durchgeführt und/oder unterstützt. Dabei können die Verfahrensschritte von oder auf ein und demselben Computer oder Computernetzwerk ausgeführt werden. Alternativ können zwei oder mehr der Verfahrensschritte auch von getrennten, aber zusammenwirkenden Computern oder Computernetzen ausgeführt werden. Beispielsweise können alle Verfahrensschritte auf einem lokalen Computer oder Server durchgeführt werden, etwa durch Nutzung des Arbeitsspeichers des lokalen Computers oder Servers. Alternativ kann das Verfahren jedoch auch ganz oder teilweise unter Verwendung dezentraler Computerressourcen, wie zum Beispiel Cloud-Computing, durchgeführt werden.

In einem weiteren Aspekt, ist ein System offenbart, wobei das System eingerichtet ist zur Durchführung des Verfahrens zur Ermittlung und Anwendung eines Korrektursignals auf eine Korrekturspule einer NMR-Apparatur zur Kompensation von zumindest einer Störung im Magnetfeld der NMR-Apparatur, wobei das System zumindest folgendes umfasst:
- einen Computer, wobei der Computer ausgelegt ist zur Durchführung der Schritte a) bis k) des Verfahrens;
- eine NMR-Apparatur, wobei die NMR-Apparatur eine Korrekturspule umfasst, wobei die Korrekturspule ausgelegt ist zur Durchführung des Schritts 1) des Verfahrens.

Für diesen Aspekt kann auf jeden weiteren Aspekt, jede Definition und/oder jede Ausführungsform wie hierin beschrieben verwiesen werden.

Das Verfahren zur Ermittlung und Anwendung eines Korrektursignals auf eine Korrekturspule einer NMR-Apparatur zur Kompensation von zumindest einer Störung im Magnetfeld der NMR-Apparatur und das System gemäß der vorliegenden Erfindung weisen verschiedene Vorteile gegenüber den aus dem Stand der Technik bekannten Verfahren und Vorrichtungen auf.

Insbesondere kann die Drift des Hauptmagnetfeldes, die durch externe und interne Faktoren verursacht wird, ermittelt und korrigiert werden, um die Qualität der NMR-Daten zu verbessern.

Weiterhin kann insbesondere die Kontinuität und Präzision der Messungen gewährleistet werden, indem spezifische Korrekturmethoden angewendet werden.

Weiterhin kann insbesondere die Analyse von Materialien und biologischen Körpern optimiert werden, indem die Integrität bzw. Stabilität des Messsignals sichergestellt wird.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, die hierdurch eingeleiteten Merkmale mit anderen Merkmalen zu kombinieren, seien es optionale oder nicht-optionale Merkmale, unangetastet bleiben.

Zusammenfassend werden, ohne Beschränkung weiterer möglicher Ausgestaltungen, folgende Ausführungsformen vorgeschlagen:
Ausführungsform 1: Verfahren zur Ermittlung und Anwendung eines Korrektursignals auf eine Korrekturspule einer NMR-Apparatur zur Kompensation von zumindest einer Störung im Magnetfeld der NMR-Apparatur, umfassend die Schritte:
   a) Unterteilung eines magnetischen Messsignals in eine Vielzahl von Teilsignalen, wobei das eine magnetische Messsignal in sich wiederholenden Störperioden gleicher Länge *T* zumindest eine Störung aufgrund der zumindest einen Störung im Magnetfeld der NMR-Apparatur erfährt, wobei jedes Teilsignal dem einen magnetischen Messsignal in einer Störperiode entspricht,
   b) Ermittlung eines Glättungssignals für jedes Teilsignal aus dem Teilsignal,
   c) Berechnung einer Standardabweichung für jedes Teilsignal von dessen Glättungssignal,
   d) Bestimmung eines Konfidenzintervalls für alle Punkte jedes Teilsignals unter Verwendung des Glättungssignals und einem k-fachen der ermittelten Standardabweichung des entsprechenden Teilsignals, wobei k eine positive rationale Zahl größer oder gleich 1 ist,
   e) Ermittlung kritischer Punkte jedes Teilsignals, wobei die kritischen Punkte die Punkte sind, die außerhalb des Konfidenzintervalls liegen,
   f) Berechnung einer korrigierten Standardabweichung wie unter Schritt c) aber mit Auslassung aller kritischen Punkte aus Schritt e),
   g) Erneute Ermittlung der kritischen Punkte jedes Teilsignals unter Berücksichtigung aller Punkte jedes Teilsignals, wobei kritische Punkte die Punkte sind, bei denen die Absolutdifferenz zwischen einem Punkt und seinem folgenden Punkt des entsprechenden Teilsignals größer als ein k-faches der korrigierten Standardabweichung ist.,
   h) Klassifizierung der kritischen Punkte in Polstellen oder Sprungstellen,
      I. wobei ein kritischer Punkt einer Polstelle entspricht, wenn die Absolutdifferenz des kritischen Punktes zu einem weiteren Punkt des entsprechenden Teilsignals in einem Suchintervall einer Breite *w* nach dem kritischen Punkt kleiner als das k-fache der korrigierten Standardabweichung ist,
      II. wobei ein kritischer Punkt einer Sprungstelle entspricht, wenn die Absolutdifferenz des kritischen Punktes zu allen Punkten des entsprechenden Teilsignals in dem Suchintervall der Breite w nach dem kritischen Punkt größer als das k-fache der korrigierten Standardabweichung ist,
   i) Korrektur aller Punkte in einem Polstellenbereich, die auf eine Polstelle folgen und im Suchintervall der Breite w eine Absolutdifferenz zum kritischen Punkt haben, die größer als das k-fache der korrigierten Standardabweichung ist, durch Interpolation mit zu dem Polstellenbereich benachbarten Werten des Teilsignals,
   j) Korrektur einer Sprungstelle durch Subtraktion einer Sprunghöhe von allen auf die Sprungstelle folgenden Werten des Teilsignals, wobei die Sprunghöhe aus den Werten des Teilsignals vor und nach der Sprungstelle ermittelt wird,
   k) Mittelung aller durch die vorhergehenden Schritte korrigierten Teilsignale, wobei ein Korrektursignal erzeugt wird,
   l) Anwendung des Korrektursignals aus Schritt k) auf eine Korrekturspule der NMR-Apparatur zur Kompensation der zumindest einen Störung im Magnetfeld der NMR-Apparatur.
Ausführungsform 2: Verfahren nach der vorhergehenden Ausführungsform, wobei, in Schritt 1), das Korrektursignal für die Korrekturspule invertiert wird, um der zumindest einen Störung im magnetischen Messsignal entgegenzuwirken.
Ausführungsform 3: Verfahren nach einer der vorhergehenden Ausführungsformen, wobei, in Schritt j), die Werte des Teilsignals innerhalb eines Sicherheitsintervalls mit einer Breite *r* um die Sprungstelle extrapoliert werden, und die Sprunghöhe aus der Differenz zwischen dem extrapolierten Teilsignal am Rand des Sicherheitsintervalls und dem Teilsignal an dieser Stelle ermittelt wird.
Ausführungsform 4: Verfahren nach der vorhergehenden Ausführungsform, wobei entweder der Teil vor oder nach dem Sprung des Teilsignals extrapoliert wird, und die Sprunghöhe aus der Differenz zwischen dem extrapolierten Teil des Teilsignals am Rand des Sicherheitsintervalls r und dem anderen Teil des Teilsignals an dieser Stelle ermittelt wird.
Ausführungsform 5: Verfahren nach einer der zwei vorhergehenden Ausführungsformen, wobei die Breite *r* des Sicherheitsintervalls kleiner ist als die Länge *T* der Störperiode, insbesondere wobei die Breite *r* zwischen 10s und 100s, bevorzugt zwischen 20s bis 80s, liegt.
Ausführungsform 6: Verfahren nach einer der vorhergehenden Ausführungsformen, wobei in Schritt h)II. beidseitig der Sprungstelle die Krümmung des Teilsignals ermittelt wird und das Teilsignal von dem Korrekturverfahren ausgeschlossen wird, wenn die Krümmung des Teilsignals an der Sprungstelle ihr Vorzeichen wechselt.
Ausführungsform 7: Verfahren nach einer der vorhergehenden Ausführungsformen, wobei das Integral des Absolutwertes jedes korrigierten Teilsignals vor Schritt k) berechnet wird und Teilsignale mit Abweichungen des Integrals vom Mittelwert aller Intergrale von mehr als zwei Standardabweichungen aller Intergrale vom Korrekturverfahren ausgeschlossen werden.
Ausführungsform 8: Verfahren nach einer der vorhergehenden Ausführungsformen, wobei vor Schritt k) die Schritte b) bis h) erneut durchgeführt werden, wobei die Teilsignale von den Schritten k) und l) ausgeschlossen werden, wenn eine Polstelle oder eine Sprungstelle im Teilsignal vorhanden ist
Ausführungsform 9: Verfahren nach einer der vorhergehenden Ausführungsformen, wobei nach Schritt a), und bevorzugt vor Schritt b), eine Offsetkorrektur für jedes Teilsignal erfolgt, so dass die Werte der letzten Punkte der Teilsignale übereinstimmen, insbesondere wobei die Offsetkorrektur durch Subtraktion des Wertes des letzten Punktes vom entsprechenden gesamten Teilsignal erfolgt.
Ausführungsform 10: Verfahren nach einer der vorhergehenden Ausführungsformen, wobei in Schritt j) nach Korrektur der Sprungstellen eine Offsetkorrektur erfolgt, wobei in einem ersten Schritt die Werte einer Geraden vom Teilsignal abgezogen werden, die durch den ersten Wert und durch Null auf Höhe des letzten Wertes verläuft, und wobei in einem zweiten Schritt die Werte einer weiteren Geraden vom Teilsignal abgezogen werden, die auf Höhe des ersten Wertes durch Null und durch den letzten Wert des Teilsignals verläuft, so dass das so korrigierte Teilsignal am Anfang und am Ende den Wert Null hat.
Ausführungsform 11: Verfahren nach einer der vorhergehenden Ausführungsformen, wobei die Länge *T* der Störperioden zwischen 250s und 5000 s ist.
Ausführungsform 12: Verfahren nach einer der vorhergehenden Ausführungsformen, wobei eine Größe des Konfidenzintervalls sich aus dem *k*-fachen der Standardabweichung bestimmt, wobei *k* zwischen 2 und 20 liegt, insbesondere wobei das Konfidenzintervall das Glättungssignal umgibt.
Ausführungsform 13: Verfahren nach einer der vorhergehenden Ausführungsformen, wobei der Faktor *k* zwischen 2 und 20 liegt.
Ausführungsform 14: Verfahren nach einer der vorhergehenden Ausführungsformen, wobei die Breite *w* des Suchintervalls kleiner ist als die Länge *T* der Störperiode, insbesondere wobei die Breite w des Suchintervalls zwischen 1s und 30s liegt.
Ausführungsform 15: Verfahren nach einer der vorhergehenden Ausführungsformen, wobei jede Messung des magnetischen Messsignals in äquidistanten Zeitschritten zwischen 150 ms und 600 ms erfolgt ist.
Ausführungsform 16: Verfahren nach einer der vorhergehenden Ausführungsformen, wobei jedes Glättungssignal durch einen gleitenden Mittelwert mit einer Fensterbreite F über jedes Teilsignal ermittelt wird, wobei die Fensterbreite *F* kleiner ist als die Länge *T* der Störperiode, insbesondere wobei die Fensterbreite *F* zwischen 10 s und 100 s groß ist.
Ausführungsform 17: Verfahren nach einer der vorhergehenden Ausführungsformen, wobei jedes Glättungssignal durch einen Tiefpass-Filter nach einer Fouriertransformation ermittelt wird.
Ausführungsform 18: Verfahren nach einer der vorhergehenden Ausführungsformen, wobei nach Schritt k), bevorzugt vor Schritt 1), erneut eine Glättung wie nach Schritt b) erfolgt.
Ausführungsform 19: Verfahren nach einer der vorhergehenden Ausführungsformen, wobei die Standardabweichung unter Berücksichtigung aller Messwerte eines Teilsignals ermittelt wird.
Ausführungsform 20: Verfahren nach einer der vorhergehenden Ausführungsformen, wobei die Standardabweichung über eine Fensterbreite *F* ermittelt wird, wobei die Fensterbreite *F* kleiner ist als die Länge *T* der Störperiode, insbesondere wobei die Fensterbreite zwischen 10 s und 100 s ist.
Ausführungsform 21: Verfahren nach einer der vorhergehenden Ausführungsformen, wobei zumindest ein Teil der Verfahrensschritte mit zumindest einem der folgenden: , einem verschiedenen *k;* einem verschiedenen Suchintervall einer Breite *w*, insbesondere mit verschiedenen Parametern *k* und *w*, wiederholt durchgeführt wird.
Ausführungsform 22: Verfahren nach der vorhergehenden Ausführungsform, wobei in einem ersten Schritt alle Polstellen mit *k =* 10 und *w* = 30 s korrigiert werden.
Ausführungsform 23: Verfahren nach der vorhergehenden Ausführungsform, wobei in einem zweiten Schritt alle Sprungstellen mit *k* = 5 und *w* = 2 s korrigiert werden.
Ausführungsform 24: Verfahren nach der vorhergehenden Ausführungsform, wobei in einem dritten Schritt verbliebene Polstellen mit *k* = 5 und *w* = 5 s korrigiert werden.
Ausführungsform 25: Verfahren nach der vorhergehenden Ausführungsform, wobei in einem vierten Schritt die Anwendung des korrigierten Teilsignals verworfen wird, wenn mit *k* = 15 und *w* = 1 s noch Polstellen oder Sprungstellen vorhanden sind.
Ausführungsform 26: Verfahren nach einer der vorhergehenden Ausführungsformen, wobei das Verfahren ein computer-implementiertes Verfahren ist,
   insbesondere wobei die Verfahrensschritte a) bis k) von einem Computer durchgeführt werden, wobei der Verfahrensschritt 1) mittels der Korrekturspule der NMR-Apparatur durchgeführt werden.
Ausführungsform 27: Ein System, wobei das System eingerichtet ist zur Durchführung des Verfahrens zur Ermittlung und Anwendung eines Korrektursignals auf eine Korrekturspule einer NMR-Apparatur zur Kompensation von zumindest einer Störung im Magnetfeld der NMR-Apparatur nach einer der vorhergehenden Ausführungsformen bezogen auf ein Verfahren, wobei das System zumindest umfasst:
   - einen Computer, wobei der Computer ausgelegt ist zur Durchführung der Schritte a) bis k) des Verfahrens;
   - eine NMR-Apparatur, wobei die NMR-Apparatur eine Korrekturspule umfasst, wobei die Korrekturspule ausgelegt ist zur Durchführung des Schritts 1) des Verfahrens.

### Kurze Beschreibung der Figuren

Weitere Einzelheiten und Merkmale ergeben sich aus der nachfolgenden Beschreibung von Ausführungsbeispielen, insbesondere in Verbindung mit den Unteransprüchen. Hierbei können die jeweiligen Merkmale für sich allein oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:
- Figur 1: ein exemplarisches Verfahren zur Korrektur eines magnetischen Messsignals mit einer NMR-Apparatur;
- Figur 2: exemplarische Messkurven verschiedener Teilsignale *H*₀(*t*)*;*
- Figuren 3a und 3b: exemplarische Messkurven verschiedener Teilsignale *H*₀(*t*) mit einer Polstelle und einer Sprungstelle;
- Figur 4: ein exemplarisches Konfidenzintervalls *H*_{0*,*±}(*t*) um einem Glättungssignal 〈*H*₀〉*_{G}*(*t*);
- Figuren 5a und 5b: eine exemplarische Korrektur einer Messkurve eines Teilsignals *H*₀(*t*) mit einer Polstelle;
- Figuren 6a und 6b: eine exemplarische Korrektur einer Messkurve eines Teilsignals *H*₀(*t*) mit einer Sprungstelle;
- Figuren 7a und 7b: exemplarische Messkurven eines Teilsignals *H*₀(*t*) mit unterschiedlichen Sprungstellen;
- Figuren 8a und 8b: ein exemplarische Offsetkorrektur einer korrigierten Messkurve eines Teilsignals *H*₀(*t*)*;*
- Figur 9: exemplarische offsetkorrigierte Messkurven von Teilsignalen *H*₀(*t*);
- Figuren 10a und 10b: exemplarische Teilsignale; und
- Figuren 11a und 11b: exemplarische geglättete Teilsignale aus den Figuren 10a und 10b.

### Beschreibung der Ausführungsbeispiele

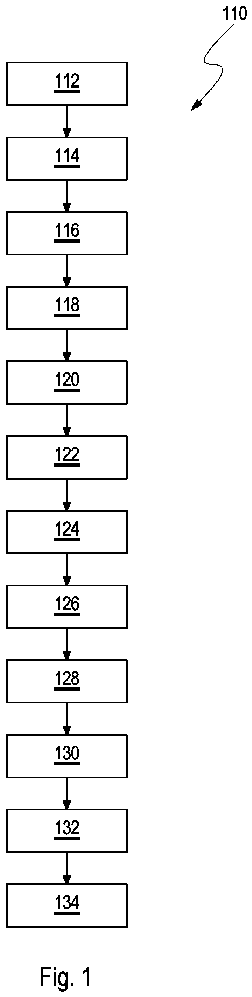

Figur 1 zeigt ein exemplarisches Verfahren 110 zur Ermittlung und Anwendung eines Korrektursignals auf eine Korrekturspule einer NMR-Apparatur zur Kompensation von zumindest einer Störung im Magnetfeld der NMR-Apparatur.

Das Verfahren umfasst die Schritte:
a) (Angegeben mit dem Bezugszeichen 112) Unterteilung eines magnetischen Messsignals in eine Vielzahl von Teilsignalen, wobei das eine magnetische Messsignal in sich wiederholenden Störperioden gleicher Länge *T* zumindest eine Störung aufgrund der zumindest einen Störung im Magnetfeld der NMR-Apparatur erfährt, wobei jedes Teilsignal dem einen magnetischen Messsignal in einer Störperiode entspricht,
b) (Angegeben mit dem Bezugszeichen 114) Ermittlung eines Glättungssignals für jedes Teilsignal aus dem Teilsignal,
c) (Angegeben mit dem Bezugszeichen 116) Berechnung einer Standardabweichung für jedes Teilsignal von dessen Glättungssignal,
d) (Angegeben mit dem Bezugszeichen 118) Bestimmung eines Konfidenzintervalls für alle Punkte jedes Teilsignals unter Verwendung des Glättungssignals und der einem k-fachen der ermittelten Standardabweichung des entsprechenden Teilsignals, wobei k eine positive rationale Zahl größer oder gleich 1 ist,
e) (Angegeben mit dem Bezugszeichen 120) Ermittlung kritischer Punkte jedes Teilsignals, wobei die kritischen Punkte die Punkte sind, die außerhalb des Konfidenzintervalls liegen,
f) (Angegeben mit dem Bezugszeichen 122) Berechnung einer korrigierten Standardabweichung wie unter Schritt c) aber mit Auslassung aller kritischen Punkte aus Schritt e),
g) (Angegeben mit dem Bezugszeichen 124) Erneute Ermittlung der kritischen Punkte jedes Teilsignals unter Berücksichtigung aller Punkte jedes Teilsignals, wobei die kritischen Punkte die Punkte sind, bei denen die Absolutdifferenz zwischen dem Punkt und dem folgenden Punkt des entsprechenden Teilsignals größer ist als ein k-faches der korrigierten Standabweichung,
h) (Angegeben mit dem Bezugszeichen 126) Klassifizierung der kritischen Punkte in Polstellen oder Sprungstellen,
   I. wobei ein kritischer Punkt einer Polstelle entspricht, wenn die Absolutdifferenz des kritischen Punktes zu einem weiteren Punkt des entsprechenden Teilsignals in einem Suchintervall einer Breite *w* nach dem kritischen Punkt kleiner als das k-fache der korrigierten Standardabweichung ist,
   II. wobei ein kritischer Punkt einer Sprungstelle entspricht, wenn die Absolutdifferenz des kritischen Punktes zu allen Punkten des entsprechenden Teilsignals in dem Suchintervall der Breite *w* nach dem kritischen Punkt größer als das k-fache der korrigierten Standardabweichung ist,
i) (Angegeben mit dem Bezugszeichen 128) Korrektur aller Punkte in einem Polstellenbereich, die auf eine Polstelle folgen und im Suchintervall der Breite w eine Absolutdifferenz zum kritischen Punkt haben, die größer als das k-fache der korrigierten Standardabweichung ist, durch Interpolation mit zu dem Polstellenbereich benachbarten Werten des Teilsignals,
j) (Angegeben mit dem Bezugszeichen 130) Korrektur einer Sprungstelle durch Subtraktion einer Sprunghöhe von allen auf die Sprungstelle folgenden Werten des Teilsignals, wobei die Sprunghöhe aus den Werten des Teilsignals vor und nach der Sprungstelle ermittelt wird,
k) (Angegeben mit dem Bezugszeichen 132) Mittelung aller durch die vorhergehenden Schritte korrigierten Teilsignale, wobei ein Korrektursignal erzeugt wird,
l) (Angegeben mit dem Bezugszeichen 134) Anwendung des Korrektursignals aus Schritt k) auf eine Korrekturspule der NMR-Apparatur zur Kompensation der zumindest einen Störung im Magnetfeld der NMR-Apparatur.

Nach Schritt a) (angegeben mit dem Bezugszeichen 112), und bevorzugt vor Schritt b) (angegeben mit dem Bezugszeichen 114), kann eine Offsetkorrektur für jedes Teilsignal erfolgen, so dass die Werte der letzten Punkte der Teilsignale übereinstimmen, insbesondere wobei die Offsetkorrektur durch Subtraktion des Wertes des letzten Punktes vom entsprechenden gesamten Teilsignal erfolgen kann.

In Schritt h)II. (angegeben mit dem Bezugszeichen 126) kann beidseitig der Sprungstelle die Krümmung des Teilsignals ermittelt werden und das Teilsignal kann von dem Korrekturverfahren ausgeschlossen werden, wenn die Krümmung des Teilsignals an der Sprungstelle ihr Vorzeichen wechselt.

In Schritt j) (angegeben mit dem Bezugszeichen 130), können die Werte des Teilsignals innerhalb eines Sicherheitsintervalls mit einer Breite *r* um die Sprungstelle extrapoliert werden, und die Sprunghöhe aus der Differenz zwischen dem extrapolierten Teilsignal am Rand des Sicherheitsintervalls und dem Teilsignal an dieser Stelle ermittelt werden. Entweder kann der Teil vor oder nach dem Sprung des Teilsignals extrapoliert werden, und die Sprunghöhe kann aus der Differenz zwischen dem extrapolierten Teil des Teilsignals am Rand des Sicherheitsintervalls *r* und dem anderen Teil des Teilsignals an dieser Stelle ermittelt werden.

In Schritt j) (angegeben mit dem Bezugszeichen 130) kann nach Korrektur der Sprungstellen eine Offsetkorrektur erfolgen, wobei in einem ersten Schritt die Werte einer Geraden vom Teilsignal abgezogen werden können, die durch den ersten Wert und durch Null auf Höhe des letzten Wertes verläuft, und wobei in einem zweiten Schritt die Werte einer weiteren Geraden vom Teilsignal abgezogen werden können, die auf Höhe des ersten Wertes durch Null und durch den letzten Wert des Teilsignals verläuft, so dass das so korrigierte Teilsignal am Anfang und am Ende den Wert Null hat.

Das Integral des Absolutwertes jedes korrigierten Teilsignals kann, insbesondere nach Schritt j) (angegeben mit dem Bezugszeichen 130) und, vor Schritt k) (angegeben mit dem Bezugszeichen 132) berechnet werden und Teilsignale mit Abweichungen des Integrals vom Mittelwert aller Intergrale von mehr als zwei Standardabweichungen aller Intergrale können vom Korrekturverfahren ausgeschlossen werden.

Vor Schritt k) (angegeben mit dem Bezugszeichen 132) können die Schritte b) (angegeben mit dem Bezugszeichen 114) bis h) (angegeben mit dem Bezugszeichen 126) erneut durchgeführt werden, wobei die Teilsignale von den Schritten k) (angegeben mit dem Bezugszeichen 132) und 1) (angegeben mit dem Bezugszeichen 134) ausgeschlossen werden können, wenn eine Polstelle oder eine Sprungstelle im Teilsignal vorhanden ist

Nach Schritt k) (angegeben mit dem Bezugszeichen 132), bevorzugt vor Schritt 1) ) (angegeben mit dem Bezugszeichen 134), kann erneut eine Glättung wie nach Schritt b) ) (angegeben mit dem Bezugszeichen 114) erfolgen.

In Schritt 1) (angegeben mit dem Bezugszeichen 134), kann das Korrektursignal für die Korrekturspule invertiert werden, um der zumindest einen Störung im magnetischen Messsignal entgegenzuwirken.

Ein Teil der Verfahrensschritte kann mit folgenden Variationen wiederholt ausgeführt werden: einem verschiedenen k; einem verschiedenen Suchintervall einer Breite *w*, insbesondere mit verschiedenen Parametern *k* und *w*. In einem ersten Schritt können alle Polstellen mit *k* = 10 und *w* = 30 s korrigiert werden. In einem zweiten Schritt können alle Sprungstellen mit *k* = 5 und *w* = 2 s korrigiert werden. In einem dritten Schritt können verbliebene Polstellen mit *k =* 5 und *w* = 5 s korrigiert werden. In einem vierten Schritt können die Anwendung des korrigierten Teilsignals verworfen werden, wenn mit *k =* 15 und *w* = 1 s noch Polstellen oder Sprungstellen vorhanden sind.

In Folge wird das Verfahren anhand weiterer Figuren illustriert.

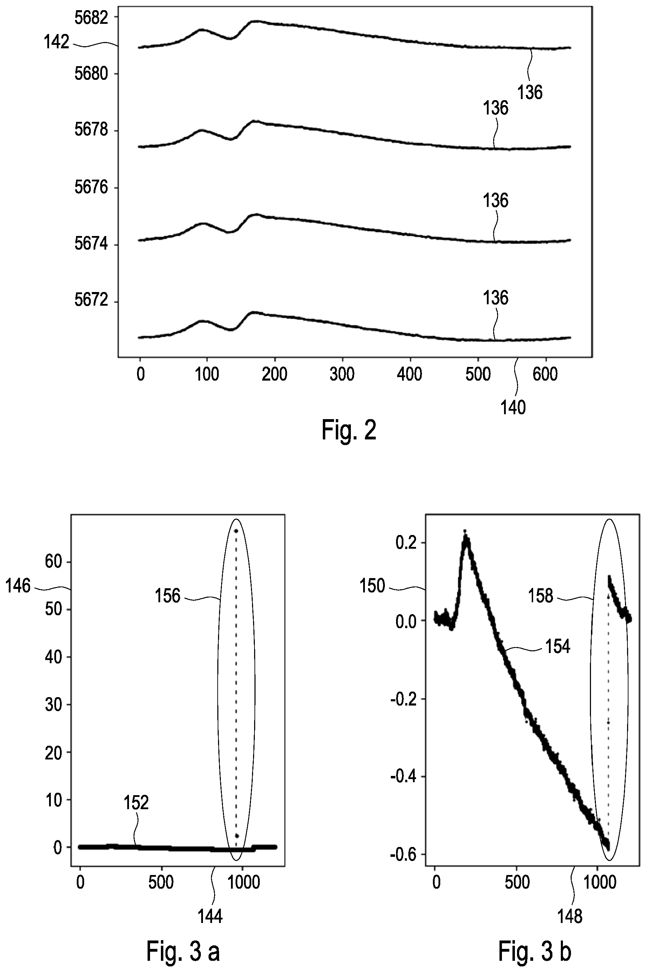

Figur 2 zeigt verschiedene exemplarische Messkurven eines Teilsignals *H*₀(*t*) (angegeben mit dem Bezugszeichen 136) in einer Störperiode, wie sie beispielsweise in Schritt a) (angegeben mit dem Bezugszeichen 112, vgl. Figur 1) generiert werden können. Auf der horizontalen Achse 140 ist die Zeit in Sekunden (s) dargestellt. Auf der vertikalen Achse 142 ist (bis auf eine Konstante) die zeitliche Variation in Hertz (Hz) der zur Stärke des angelegten Magnetfeldes proportionalen Resonanzfrequenz der Kernspins einer NMR-Probe dargestellt.

Die Figur 2 zeigt mehrere Messkurven von Teilsignalen *H*₀(*t*) (angegeben mit dem Bezugszeichen 136), die geringfügig variierende vertikale Verschiebungen aufweisen. Diese Verschiebungen beruhen auf typischen Änderungen in einer NMR-Apparatur, die in der Folge des Ladens des Magneten auftreten können, sowie auf einer Subtraktion einer linearen Basislinie zur Entfernung transienter Netto-Variationen. Abgesehen von diesen Verschiebungen weisen alle Messkurven die gleiche charakteristische Störung auf. Diese unerwünschte zeitliche Variation wird durch die vorliegende Erfindung kompensiert.

Um sicherzustellen, dass die Kompensation der Störung keine periodische absolute Änderung darstellt, können die Messkurven der Teilsignale *H*₀(*t*) (angegeben mit dem Bezugszeichen 136) zunächst um einen konstanten asymptotischen Wert für große Zeiten entlang der y-Achse verschoben werden, so dass alle Messkurven der Teilsignale *H*₀(*t*) (angegeben mit dem Bezugszeichen 136) für die weitere Analyse asymptotisch gegen Null gehen. Dadurch wird die Vergleichbarkeit und Genauigkeit der Messungen verbessert.

Wie bereits erläutert, kann dies dadurch erreicht werden, dass nach Schritt a) (angegeben mit dem Bezugszeichen 112), und bevorzugt vor Schritt b) (angegeben mit dem Bezugszeichen 114), eine Offsetkorrektur für jedes Teilsignal erfolgt, so dass die Werte der letzten Punkte der Teilsignale übereinstimmen. Bevorzugt kann die Offsetkorrektur durch Subtraktion des Wertes des letzten Punktes vom entsprechenden gesamten Teilsignal erfolgen.

Die auf diese Weise erhaltenen Messkurven der Teilsignale *H*₀(*t*) können diverse Störungen enthalten, die beispielsweise durch elektrische Störsignale, mechanische Vorgänge im Magneten, Umwelteinflüsse oder menschliche Faktoren verursacht werden. Das Vorhandensein solcher Störungen kann die weitere Verarbeitung der Messkurven der Teilsignale *H*₀(*t*) *zu*nächst erschweren. Daher kann bei jeder Messkurven der Teilsignale *H*₀(*t*) überprüft werden, ob sie Störungen enthält. Dies kann automatisch erfolgen. Falls Störungen vorhanden sind, kann entweder versucht werden, diese zu beheben, oder, falls dies nicht gelingt, können einzelne Messkurven der Teilsignale *H*₀(*t*) verworfen werden. Dies bedeutet, dass sie nicht für Ermittlung der Kompensation der Störung weiter herangezogen werden, sondern nur Messkurven anderer Teilsignale. Die Störungen können grob in zwei Kategorien eingeteilt werden: Polstellen, die virtuelle Feldänderungen darstellen, und Sprungstellen, die reale Feldänderungen darstellen. Diese verschiedenen Kategorien sind in Figur 3a und 3b veranschaulicht.

Figur 3a zeigt eine Messkurve eines Teilsignals *H*₀(*t*) (angegeben mit dem Bezugszeichen 152). Die Messkurve des Teilsignals *H*₀(*t*) (angegeben mit dem Bezugszeichen 152) zeigt eine Polstelle, die mittels der Ellipse 156 markiert ist. Auch Figur 3b zeigt eine Messkurve eines Teilsignals *H*₀(*t*) (angegeben mit dem Bezugszeichen 154). Die Messkurve des Teilsignals *H*₀(*t*) (angegeben mit dem Bezugszeichen 154) zeigt eine Sprungstelle, die mittels der Ellipse 158 markiert ist. Auf den horizontalen Achsen 144, 148 der Figuren 3a und 3b ist jeweils die Zeit in Sekunden (s) dargestellt. Auf den vertikalen Achsen 146, 150 ist jeweils (bis auf eine Konstante) die zeitliche Variation in Hertz (Hz) der zur Stärke des angelegten Magnetfeldes proportionalen Resonanzfrequenz der Kernspins einer NMR-Probe dargestellt.

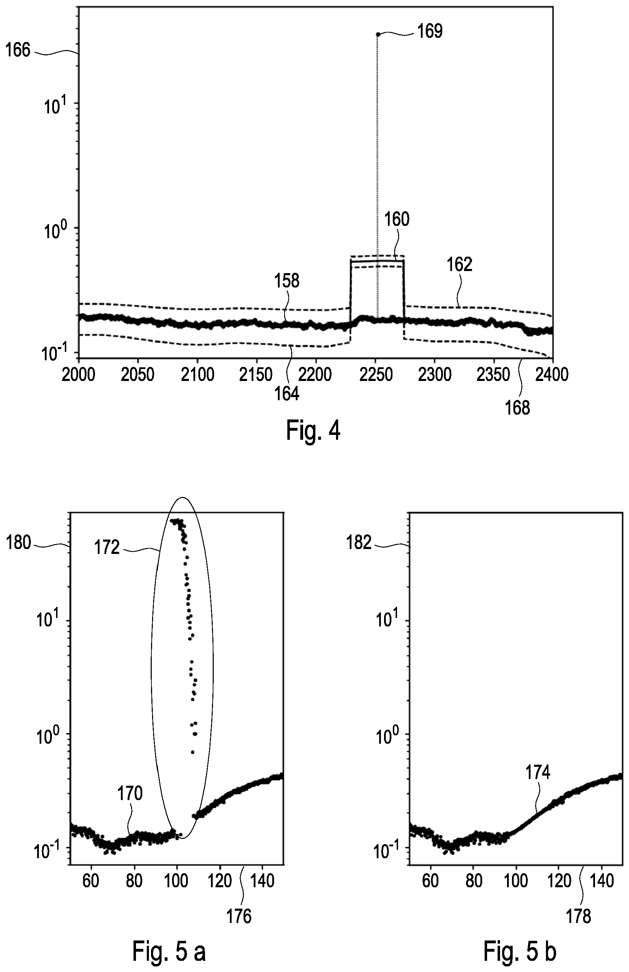

Figur 4 zeigt eine Messkurve eines Teilsignals *H*₀(*t*) (angegeben mit dem Bezugszeichen 158). Weiterhin ist in das Glättungssignal 〈*H*₀〉*_{G}*(*t*) (angegeben mit dem Bezugszeichen 160) dargestellt. Zudem sind auch die obere Grenze *H*_{0*,*±}(*t*) (angegeben mit dem Bezugszeichen 162) und die untere Grenze *H*_{0*,-*}(*t*) (angegeben mit dem Bezugszeichen 164) des Konfidenzintervalls *H*_{0*,*±}(*t*) dargestellt. Auf der horizontalen Achse 168 ist die Zeit in Sekunden (s) dargestellt. Auf der vertikalen Achse 166 ist (bis auf eine Konstante) die zeitliche Variation in Hertz (Hz) der zur Stärke des angelegten Magnetfeldes proportionalen Resonanzfrequenz der Kernspins einer NMR-Probe dargestellt, wobei zur besseren Sichtbarkeit der Daten und des Konfidenzintervalls im Verhältnis zur Polstelle eine logarithmische Achsenskalierung gewählt wurde. Der Punkt 169 liegt nicht innerhalb des Konfidenzintervalls *H*_{0*,*±}(*t*)*,* vielmehr liegt hier eine Polstelle vor.

Figur 5a zeigt eine Messkurve eines Teilsignals *H*₀(*t*) (angegeben mit dem Bezugszeichen 170). Die Messkurve eines Teilsignals *H*₀(*t*) (angegeben mit dem Bezugszeichen 170) weist eine breite Polstelle (einen Polstellenbereich) auf, die mittels der Ellipse 172 kenntlich gemacht ist. Figur 5b zeigt eine korrigierte Messkurve eines Teilsignals *H*₀(*t*) (angegeben mit dem Bezugszeichen 174), die mittels des zuvor beschriebenen Verfahrens korrigiert wurde. Bei der korrigierte Messkurve eines Teilsignals *H*₀(*t*) (angegeben mit dem Bezugszeichen 174) wurden die Polstelle behoben und die Messkurve eines Teilsignals *H*₀(*t*) im Polstellenbereich interpoliert. Auf den horizontalen Achsen 176, 178 der Figuren 5a und 5b ist jeweils die Zeit in Sekunden (s) dargestellt. Auf den vertikalen Achsen 180, 182 ist jeweils (bis auf eine Konstante) die zeitliche Variation in Hertz (Hz) der zur Stärke des angelegten Magnetfeldes proportionalen Resonanzfrequenz der Kernspins einer NMR-Probe in logarithmischer Skala dargestellt.

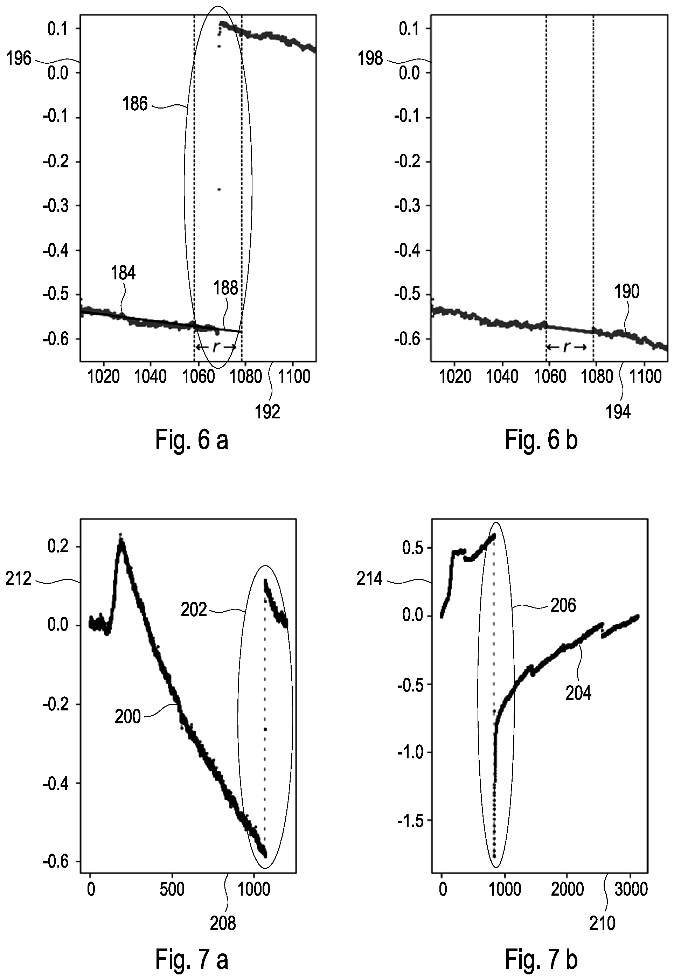

Figur 6a zeigt eine Messkurve eines Teilsignals *H*₀(*t*) (angegeben mit dem Bezugszeichen 184). Die Messkurve eines Teilsignals *H*₀(*t*) (angegeben mit dem Bezugszeichen 184) weist eine Sprungstelle auf, die mittels der Ellipse 186 kenntlich gemacht ist. Um die Sprungstelle zu korrigieren, wurde die Messkurve des Teilsignals *H*₀(*t*) (angegeben mit dem Bezugszeichen 184) vor der Sprungstelle extrapoliert. Figur 6a zeigt weiterhin die extrapolierte Messkurve des Teilsignals 188. Weiterhin ist die Breite r innerhalb der extrapoliert wurde dargestellt. Figur 6b zeigt die korrigierte Messkurve des Teilsignals *H*₀(*t*) (angegeben mit dem Bezugszeichen 190), die mittels des zuvor beschriebenen Verfahrens korrigiert wurde. Auf den horizontalen Achsen 192, 194 der Figuren 6a und 6b ist jeweils die Zeit in Sekunden (s) dargestellt. Auf den vertikalen Achsen 196, 198 ist jeweils (bis auf eine Konstante) die zeitliche Variation in Hertz (Hz) der zu Stärke des angelegten Magnetfeldes proportionalen Resonanzfrequenz der Kernspins einer NMR-Probe dargestellt. Die vertikalen Achsenabschnitte 196, 198 sind hier so gewählt, dass der Sprung in Figur 6a gut sichtbar ist und die Darstellung des korrigierten Sprungs in Figur 6b den gleichen Maßstab hat.

Wie bereits dargelegt, kann in Schritt h)II. (angegeben mit dem Bezugszeichen 126 in Figur 1) beidseitig der Sprungstelle die Krümmung des Teilsignals ermittelt werden und das Teilsignal kann von dem Korrekturverfahren ausgeschlossen werden, wenn die Krümmung des Teilsignals an der Sprungstelle ihr Vorzeichen wechselt.

Figur 7a zeigt eine Messkurve eines Teilsignals *H*₀(*t*) (angegeben mit dem Bezugszeichen 200). Die Messkurve eines Teilsignals *H*₀(*t*) (angegeben mit dem Bezugszeichen 200) weist eine Sprungstelle auf, die mittels der Ellipse 202 kenntlich gemacht ist. Die Messkurve des Teilsignals *H*₀(*t*) (angegeben mit dem Bezugszeichen 200) zeigt vor und nach der Sprungstelle jeweils eine Krümmung mit dem gleichen Vorzeichen. Die Messkurve dieses Teilsignals *H*₀(*t*) (angegeben mit dem Bezugszeichen 200) wird nicht vom Korrekturverfahren ausgeschlossen.

Figur 7b zeigt eine weitere Messkurve eines Teilsignals *H*₀(*t*) (angegeben mit dem Bezugszeichen 204). Die Messkurve des Teilsignals *H*₀(*t*) (angegeben mit dem Bezugszeichen 204) weist eine andere Sprungstelle auf, die mittels der Ellipse 206 kenntlich gemacht ist. Die Messkurve eines Teilsignals *H*₀(*t*) (angegeben mit dem Bezugszeichen 204) zeigt vor und nach der Sprungstelle jeweils eine Krümmung mit einem verschiedenen Vorzeichen. Die Messkurve dieses Teilsignals *H*₀(*t*) (angegeben mit dem Bezugszeichen 204) wird vom Korrekturverfahren ausgeschlossen.

Auf den horizontalen Achsen 208, 210 der Figuren 7a und 7b ist jeweils die Zeit in Sekunden (s) dargestellt. Auf den vertikalen Achsen 212, 214 ist jeweils (bis auf eine Konstante) die zeitliche Variation in Hertz (Hz) der zur Stärke des angelegten Magnetfeldes proportionalen Resonanzfrequenz der Kernspins einer NMR-Probe dargestellt. Die vertikalen Achsenabschnitte 212, 214 sind hier so gewählt, dass die beiden verschiedenen Sprungarten in Figur 7a und Figur 7b jeweils formatfüllend dargestellt werden.

Wie bereits dargelegt kann in Schritt j) (angegeben mit dem Bezugszeichen 130 in Figur 1) nach Korrektur der Sprungstellen eine Offsetkorrektur erfolgen, wobei in einem ersten Schritt die Werte einer Geraden vom Teilsignal abgezogen werden, die durch den ersten Wert und durch Null auf Höhe des letzten Wertes verläuft. In einem zweiten Schritt können die Werte einer weiteren Geraden vom Teilsignal abgezogen werden, die auf Höhe des ersten Wertes durch Null und durch den letzten Wert des Teilsignals verläuft, so dass das so korrigierte Teilsignal am Anfang und am Ende den Wert Null hat.

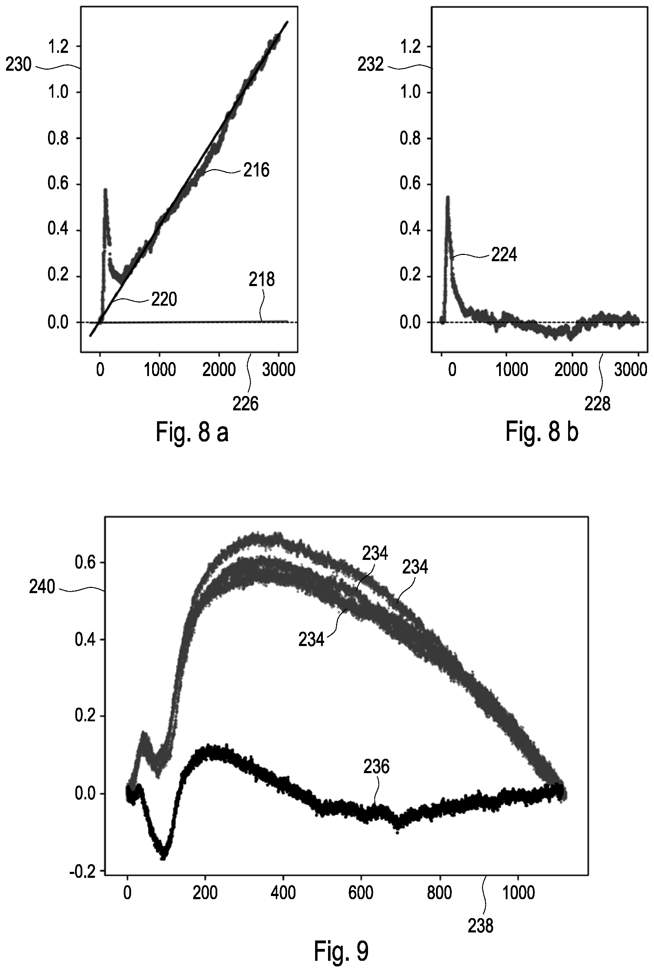

Figur 8a zeigt eine korrigierte Messkurve eines Teilsignals *H*₀(*t*) (angegeben mit dem Bezugszeichen 216) nach Korrektur der Sprungstellen. Weiterhin sind eine Gerade 218, die in dem ersten Schritt generiert wurde, und eine Gerade 220, die in dem zweiten Schritt generiert wurde, dargestellt. Mittels der Geraden 218, 220 wird die Offsetkorrektur durchgeführt. Figur 8b zeigt eine entsprechend offsetkorrigierte Messkurve eines Teilsignals *H*₀(*t*) (angegeben mit dem Bezugszeichen 224) nach Korrektur der Sprungstellen.

Auf den horizontalen Achsen 226, 228 der Figuren 8a und 8b ist jeweils die Zeit in Sekunden (s) dargestellt. Auf den vertikalen Achsen 230, 232 ist jeweils (bis auf eine Konstante) die zeitliche Variation in Hertz (Hz) der zur Stärke des angelegten Magnetfeldes proportionalen Resonanzfrequenz der Kernspins einer NMR-Probe dargestellt.

Wie bereits dargelegt kann das Integral des Absolutwertes jedes korrigierten Teilsignals vor Schritt k) (angegeben mit dem Bezugszeichen 132 in Figur 1), und insbesondere nach Schritt j) (angegeben mit dem Bezugszeichen 130 in Figur 1), berechnet werden und Teilsignale mit Abweichungen des Integrals vom Mittelwert aller Intergrale von mehr als zwei Standardabweichungen aller Intergrale können vom Korrekturverfahren ausgeschlossen werden.

Figur 9 zeigt korrigierte Messkurven mehrerer Teilsignale *H*₀(*t*) (angegeben mit den Bezugszeichen 234, 236) nach Korrektur der Sprungstellen und/oder Polstellen. Eine der Messkurven der Teilsignale *H*₀(*t*) (angegeben mit dem Bezugszeichen 236) hat ein Absolutwert eines Integrals, der mehr als zwei Standardabweichungen vom Mittelwert aller Absolutwerte der Integrale der Messkurven der Teilsignale *H*₀(*t*) (angegeben mit dem Bezugszeichen 234, 236) abweicht. Die Messkurve des Teilsignals *H*₀(*t*) (angegeben mit dem Bezugszeichen 236) kann insofern vom Messverfahren ausgeschlossen werden. Auf der horizontalen Achse 238 ist die Zeit in Sekunden (s) dargestellt. Auf der vertikalen Achse 240 ist (bis auf eine e Konstante) die zeitliche Variation in Hertz (Hz) der zur Stärke des angelegten Magnetfeldes proportionalen Resonanzfrequenz der Kernspins einer NMR-Probe dargestellt.

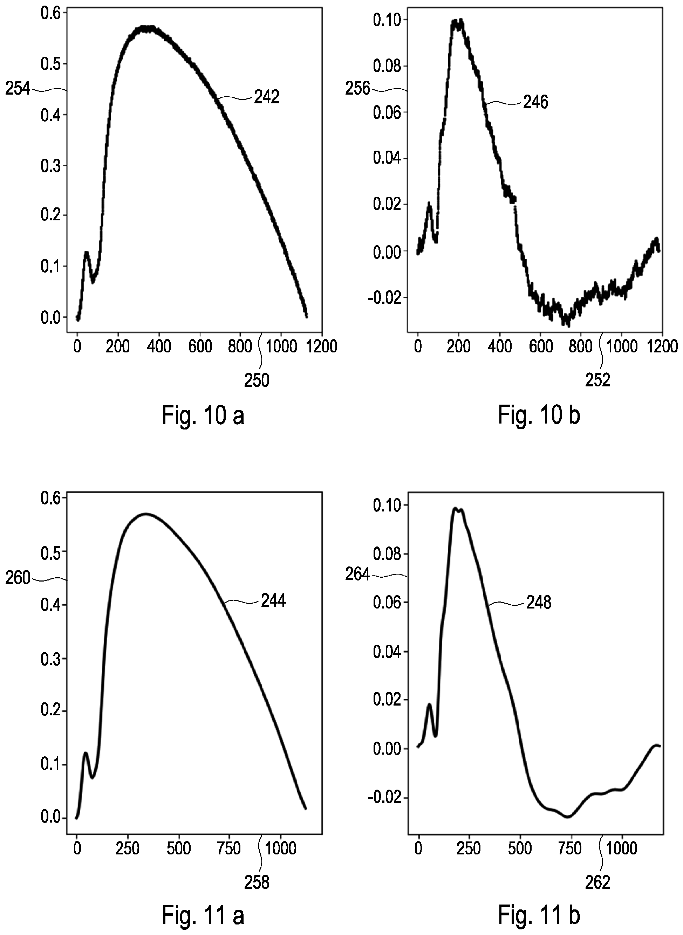

Wie bereits dargelegt kann nach Schritt k) (angegeben mit dem Bezugszeichen 132), bevorzugt vor Schritt 1) (angegeben mit dem Bezugszeichen 134), erneut eine Glättung wie nach Schritt b) (angegeben mit dem Bezugszeichen 114) erfolgen. Figur 10a zeigt eine korrigierte Messkurve eines Teilsignals *H*₀(*t*) (angegeben mit dem Bezugszeichen 242). Auch Figur 10b zeigt eine weitere korrigierte Messkurve eines Teilsignals *H*₀(*t*) (angegeben mit dem Bezugszeichen 246) Figur 11a zeigt ein geglättetes Teilsignal 244 der Messkurve aus Figur 10a. Figur 11b zeigt ein geglättetes Teilsignal 248 der Messkurve aus Figur 10b. Die weitere korrigierte Messkurve eines Teilsignals *H*₀(*t*) (angegeben mit dem Bezugszeichen 246) weist ein stärkeres Rauschen als das der korrigierten Messkurve des Teilsignals *H*₀(*t*) (angegeben mit dem Bezugszeichen 242) auf. Auf den horizontalen Achsen 250, 252, 258 und 262 der Figuren 10a, 10b, 11a und 11b ist jeweils die Zeit in Sekunden (s) dargestellt. Auf den vertikalen Achsen 254, 256, 260 und 264 ist jeweils (bis auf eine Konstante) die zeitliche Variation in Hertz (Hz) der zur Stärke des angelegten Magnetfeldes proportionalen Resonanzfrequenz der Kernspins einer NMR-Probe dargestellt.

### Bezugszeichenliste

- 110: Verfahren zur Korrektur eines magnetischen Messsignals
- 112: Unterteilung des einen magnetischen Messsignals
- 114: Ermittlung eines Glättungssignals für jedes Teilsignal aus dem Teilsignal
- 116: Berechnung einer Standardabweichung für jedes Teilsignal
- 118: Bestimmung eines Konfidenzintervalls
- 120: Ermittlung kritischer Punkte jedes Teilsignals
- 122: Berechnung einer korrigierten Standardabweichung
- 124: Erneute Ermittlung der kritischen Punkte
- 126: Klassifizierung der kritischen Punkte in Polstellen oder Sprungstellen
- 128: Korrektur einer oder mehrerer zusammenhängender Polstellen
- 130: Korrektur einer Sprungstelle
- 132: Mittelung aller durch die vorhergehenden Schritte korrigierten Teilsignale
- 134: Anwendung des Korrektursignals
- 136: exemplarische Messkurven eines Teilsignals *H*₀(*t*)
- 140: horizontalen Achse
- 142: vertikale Achse
- 144: horizontale Achse
- 146: vertikale Achse
- 148: horizontale Achse
- 150: vertikale Achse
- 152: Messkurve eines Teilsignals *H*₀(*t*)
- 154: Messkurve eines Teilsignals *H*₀(*t*)
- 156: Polstelle
- 158: Messkurve eines Teilsignals *H*₀(*t*)
- 160: Glättungssignal 〈*H*₀〉*_{G}*(*t*)
- 162: obere Grenze *H*_{0*,+*}(*t*) des Konfidenzintervalls *H*_{0*,*±}(*t*)
- 164: untere Grenze *H*_{0*,-*}(*t*) des Konfidenzintervalls *H*_{0*,*±}(*t*)
- 166: vertikale Achsen
- 168: horizontale Achse
- 169: Punkt außerhalb des Konfidenzintervalls *H*_{0*,*±}(*t*)
- 170: Messkurve eines Teilsignals *H*₀(*t*)
- 172: breite Polstelle
- 174: korrigierte Messkurve eines Teilsignals *H*₀(*t*)
- 176: horizontale Achse
- 178: horizontale Achse
- 180: vertikale Achse
- 182: vertikale Achse
- 184: Messkurve eines Teilsignals *H*₀(*t*)
- 186: Sprungstelle
- 188: extrapolierte Messkurve des Teilsignals
- 190: korrigierte Messkurve des Teilsignals *H*₀(*t*)
- 192: horizontale Achse
- 194: horizontale Achse
- 196: vertikale Achse
- 198: vertikale Achse
- 200: Messkurve eines Teilsignals *H*₀(*t*)
- 202: Sprungstelle
- 204: Messkurve eines Teilsignals *H*₀(*t*)
- 206: Sprungstelle
- 208: horizontale Achse
- 210: horizontale Achse
- 212: vertikale Achse
- 214: vertikale Achse
- 216: Messkurve eines Teilsignals *H*₀(*t*)
- 218: Gerade
- 220: Gerade
- 224: offsetkorrigierte Messkurve eines Teilsignals *H*₀(*t*)
- 226: horizontale Achse
- 228: horizontale Achse
- 230: vertikale Achse
- 232: vertikale Achse
- 234: Messkurve eines Teilsignals *H*₀(*t*)
- 236: Messkurve eines Teilsignals *H*₀(*t*)
- 238: horizontale Achse
- 240: vertikale Achse
- 242: Messkurve eines Teilsignals *H*₀(*t*)
- 244: geglättetes Teilsignal
- 246: Messkurve eines Teilsignals *H*₀(*t*)
- 248: geglättetes Teilsignal
- 250: horizontale Achse
- 252: horizontale Achse
- 254: vertikale Achse
- 256: vertikale Achse
- 258: horizontale Achse
- 260: vertikale Achse
- 262: horizontale Achse
- 264: vertikale Achse

## Patentansprüche

1. Verfahren zur Ermittlung und Anwendung eines Korrektursignals auf eine Korrekturspule einer NMR-Apparatur zur Kompensation von zumindest einer Störung im Magnetfeld der NMR-Apparatur, umfassend die Schritte:
a) Unterteilung eines magnetischen Messsignals in eine Vielzahl von Teilsignalen, wobei das eine magnetische Messsignal in sich wiederholenden Störperioden gleicher Länge *T* zumindest eine Störung aufgrund der zumindest einen Störung im Magnetfeld der NMR-Apparatur erfährt, wobei jedes Teilsignal dem einen magnetischen Messsignal in einer Störperiode entspricht,
b) Ermittlung eines Glättungssignals für jedes Teilsignal aus dem Teilsignal,
c) Berechnung einer Standardabweichung für jedes Teilsignal von dessen Glättungssignal,
d) Bestimmung eines Konfidenzintervalls für alle Punkte jedes Teilsignals unter Verwendung des Glättungssignals und einem k-fachen der ermittelten Standardabweichung des entsprechenden Teilsignals, wobei k eine positive rationale Zahl größer oder gleich 1 ist,
e) Ermittlung kritischer Punkte jedes Teilsignals, wobei die kritischen Punkte die Punkte sind, die außerhalb des Konfidenzintervalls liegen,
f) Berechnung einer korrigierten Standardabweichung wie unter Schritt c) aber mit Auslassung aller kritischen Punkte aus Schritt e),
g) Erneute Ermittlung der kritischen Punkte jedes Teilsignals unter Berücksichtigung aller Punkte jedes Teilsignals, wobei die kritischen Punkte die Punkte sind, bei denen die Absolutdifferenz zwischen dem Punkt und dem folgenden Punkt des entsprechenden Teilsignals größer ist als das k-fache der korrigierten Standabweichung,
h) Klassifizierung der kritischen Punkte in Polstellen oder Sprungstellen,
I. wobei ein kritischer Punkt einer Polstelle entspricht, wenn die Absolutdifferenz des kritischen Punktes zu einem weiteren Punkt des entsprechenden Teilsignals in einem Suchintervall einer Breite w nach dem kritischen Punkt kleiner als das k-fache der korrigierten Standardabweichung ist,
II. wobei ein kritischer Punkt einer Sprungstelle entspricht, wenn die Absolutdifferenz des kritischen Punktes zu allen Punkten des entsprechenden Teilsignals in dem Suchintervall der Breite *w* nach dem kritischen Punkt größer als das k-fache der korrigierten Standardabweichung ist,
i) Korrektur aller Punkte in einem Polstellenbereich, die auf eine Polstelle folgen und im Suchintervall der Breite w eine Absolutdifferenz zum kritischen Punkt haben, die größer als das k-fache der korrigierten Standardabweichung ist, durch Interpolation mit zu dem Polstellenbereich benachbarten Werten des Teilsignals,
j) Korrektur einer Sprungstelle durch Subtraktion einer Sprunghöhe von allen auf die Sprungstelle folgenden Werten des Teilsignals, wobei die Sprunghöhe aus den Werten des Teilsignals vor und nach der Sprungstelle ermittelt wird,
k) Mittelung aller durch die vorhergehenden Schritte korrigierten Teilsignale, wobei ein Korrektursignal erzeugt wird,
l) Anwendung des Korrektursignals aus Schritt k) auf eine Korrekturspule der NMR-Apparatur zur Kompensation der zumindest einen Störung im Magnetfeld der NMR-Apparatur.

2. Verfahren nach dem vorhergehenden Anspruch, wobei, in Schritt 1), das Korrektursignal für die Korrekturspule invertiert wird, um der zumindest einen Störung im magnetischen Messsignal entgegenzuwirken.

3. Verfahren nach einem der vorhergehenden Ansprüche, wobei, in Schritt j), die Werte des Teilsignals innerhalb eines Sicherheitsintervalls mit einer Breite *r* um die Sprungstelle extrapoliert werden, und die Sprunghöhe aus der Differenz zwischen dem extrapolierten Teilsignal am Rand des Sicherheitsintervalls und dem Teilsignal an dieser Stelle ermittelt wird.

4. Verfahren nach einem der vorhergehenden Ansprüche, wobei in Schritt h)II. beidseitig der Sprungstelle die Krümmung des Teilsignals ermittelt wird und das Teilsignal von dem Korrekturverfahren ausgeschlossen wird, wenn die Krümmung des Teilsignals an der Sprungstelle ihr Vorzeichen wechselt.

5. Verfahren nach einem der vorhergehenden Ansprüche, wobei das Integral des Absolutwertes jedes korrigierten Teilsignals vor Schritt k) berechnet wird und Teilsignale mit Abweichungen des Integrals vom Mittelwert aller Intergrale von mehr als zwei Standardabweichungen aller Intergrale vom Korrekturverfahren ausgeschlossen werden.

6. Verfahren nach einem der vorhergehenden Ansprüche, wobei vor Schritt k) die Schritte b) bis h) erneut durchgeführt werden, wobei die Teilsignale von den Schritten k) und l) ausgeschlossen werden, wenn eine Polstelle oder eine Sprungstelle im Teilsignal vorhanden ist.

7. Verfahren nach einem der vorhergehenden Ansprüche, wobei nach Schritt a) eine Offsetkorrektur für jedes Teilsignal erfolgt, so dass die Werte der letzten Punkte der Teilsignale übereinstimmen, insbesondere wobei die Offsetkorrektur durch Subtraktion des Wertes des letzten Punktes vom entsprechenden gesamten Teilsignal erfolgt.

8. Verfahren nach einem der vorhergehenden Ansprüche, wobei in Schritt j) nach Korrektur der Sprungstellen eine Offsetkorrektur erfolgt, wobei in einem ersten Schritt die Werte einer Geraden vom Teilsignal abgezogen werden, die durch den ersten Wert und durch Null auf Höhe des letzten Wertes verläuft, und wobei in einem zweiten Schritt die Werte einer weiteren Geraden vom Teilsignal abgezogen werden, die auf Höhe des ersten Wertes durch Null und durch den letzten Wert des Teilsignals verläuft, so dass das so korrigierte Teilsignal am Anfang und am Ende den Wert Null hat.

9. Verfahren nach einem der vorhergehenden Ansprüche, wobei zumindest ein Teil der Verfahrensschritte mit zumindest einem der folgenden: einem verschiedenen k; einem verschiedenen Suchintervall einer Breite w wiederholt durchgeführt wird.

10. Verfahren nach einem der vorhergehenden Ansprüche, wobei jede Messung des magnetischen Messsignals in äquidistanten Zeitschritten zwischen 150 ms und 600 ms erfolgt ist.
